(19) **Europäisches Patentamt / European Patent Office / Office européen des brevets**

(11) **EP 3 533 456 A1**

(12) **EUROPEAN PATENT APPLICATION**
published in accordance with Art. 153(4) EPC

(43) Date of publication:
04.09.2019 Bulletin 2019/36

(21) Application number: 17863941.5

(22) Date of filing: 26.10.2017

(51) Int Cl.:
*A61K 38/14* (2006.01)  *A61P 25/22* (2006.01)

(86) International application number:
PCT/JP2017/038597

(87) International publication number:
WO 2018/079631 (03.05.2018 Gazette 2018/18)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 28.10.2016 JP 2016211733

(71) Applicant: Morinaga Milk Industry Co., Ltd.
Minato-ku
Tokyo 108-8384 (JP)

(72) Inventors:
• TAKEUCHI Takashi
Tottori-shi
Tottori 680-8550 (JP)
• MIYAKAWA Momoko
Zama-shi
Kanagawa 252-8583 (JP)
• WAKABAYASHI Hiroyuki
Zama-shi
Kanagawa 252-8583 (JP)

(74) Representative: Vossius & Partner
Patentanwälte Rechtsanwälte mbB
Siebertstrasse 3
81675 München (DE)

(54) **PREVENTIVE AGENT FOR A GROWING CHILD'S ANXIETY ATTRIBUTABLE TO STRESS IN INFANCY**

(57) Provided is an agent for preventing an anxiety-related behavior in a growing stage attributable to a stress in infancy via ingestion by an infant who receives the stress in infancy. Also provided is an agent for preventing an anxiety-related behavior in a growing stage attributable to a stress in infancy comprising a lactoferrin and/or a lactoferrin degradation product as active ingredient or a food composition for preventing an anxiety-related behavior in a growing stage attributable to a stress in infancy comprising a lactoferrin and/or a lactoferrin degradation product as active ingredient characterized in that the lactoferrin and/or the lactoferrin degradation product is used via ingestion during infancy for a normal infant at birth and receives the stress in infancy.

EP 3 533 456 A1

**Description**

Technical Field

The present technology relates to an agent for preventing an anxiety-related behavior in a growing stage attributable to a stress in infancy and a food composition for preventing an anxiety-related behavior in a growing stage attributable to a stress in infancy.

Background Art

It is becoming well known in these days that the stress in infancy influences the anxiety-related behavior in the growing stage in future. The stress in infancy may for example be a lack of a sense, basic trust, that the infant himself is loved and protected by surrounding humans, for example, guardians and childcare givers. This stress in infancy is caused sometimes by a breakup of the relationship with a human who is in contact with the infant, for example, guardians and childcare givers. This breakup of the relationship includes abandonment of a child and child neglect, which are attributable to poor family relationship such as parents' divorce, domestic violence and family member's disease and economical difficulty. The relevance between the human stress in infancy and the anxiety-related behavior observed afterward has been getting attention.

In researches of this relevance, a "maternal separation model" of rodent animals in which the neonatal animal before weaning are separated from a maternal animal is employed widely as an animal model of the stress during infancy. Based on the results of this "maternal separation model", it was reported that the anxiety-related behavior in the growing stage is derived from the maternal separation in infancy in Non-Patent Documents 1 and 2.

It was reported in Non-Patent Documents 1 and 2 that the anxiety-related behavior induced by the maternal separation was ameliorated by treating the neonatal animal which had been subjected to the maternal separation with an anxiolytic agent over several weeks during the growing stage in an animal experiment using the maternal separation model with regard to the maternal separation which is one of the stresses in infancy. As an anxiolytic agent, ethical drugs, paroxetine hydrochloride hydrate is used in Non-Patent Document 1 while thianeptine is used in Non-Patent Document 2.

It was also reported in Non-Patent Document 3 that the anxiety-related behavior could not be prevented even when a food material having anxiolytic effect was administered during the growing stage over several weeks in an animal experiment using the maternal separation model. In Non-Patent Document 3, Lactobacillus Plantarum PS128 was employed.

Citation List

Patent Documents

Patent Document 1: Japanese Patent No. 5427713
Patent Document 2: JP-A No. 2016-121109
Patent Document 3: WO No. 2014/099134
Patent Document 4: US No. 2015/0119322

Non-Patent Documents

Non-Patent Document 1: Huot R L et al., Psychopharmacology, 2001; 158: 366-373
Non-Patent Document 2: Trujillo V et al., Stress, 2016; 19(1): 91-103
Non-Patent Document 3: Liu Y W et al., Brain Research, 2016; 1631: 1-12.
Non-Patent Document 4: Semple B D et al., Prog Neurobiol, 2013; 106-107: 1-16.
Non-Patent Document 5: Geva R et al., Pediatrics, 2006; 118(1):91-100.
Non-Patent Document 6: Cosmi E et al., J Pregnancy, 2011; 364381.
Non-Patent Document 7: Higgins D J et al., Aggress Violent Behav, 2001; 6(6):547-578.
Non-Patent Document 8: Heim C et al., Biol Psychiatry, 2001; 49(12):1023-1039.
Non-Patent Document 9: Green J G et al., Arch Gen Psychiatry, 2010; 67(2):113-123.

Summary of the Invention

Technical Problem

Although there is a report of amelioration of the anxiety-related behavior by administration of the anxiolytic agent in the growing stage, there is no report that administration of a food material within the period of infancy can prevent the anxiety-related behavior which develops subsequently in the growing stage.

Accordingly, a major object of the present technology is to provide an agent for preventing the anxiety-related behavior in the growing stage attributable to the stress in infancy by ingesting it by the infant received the stress in infancy.

Solution to Problems

A substance having side effects which are as low as possible is preferable when administered to an infant. Because it is generally, however, difficult to obtain a potent efficacy when using a substance having low side effects, it was very difficult for the inventors to assume which substance is appropriate.

As a result, the inventors discovered that the anxiety-related behavior in the growing stage attributable to the stress in infancy can be prevented by ingesting during infancy the lactoferrin and/or the lactoferrin degradation product utilized as a food material for a normal infant at birth and receives the stress in infancy, and thus accomplishing the present invention.

Patent Document 1 discloses a drug against positive symptoms of schizophrenia comprising the lactoferrin as active ingredient. Patent Document 2 discloses an epidermal basement membrane protecting agent comprising a lactoferrin hydrolysate as active ingredient.

Patent Document 3 discloses a method for modulating a psychological stress for a child, which comprises administration of a milk-based nutritional composition comprising a lactoferrin derived from a non-human source to the child.

Patent Document 4 discloses a method for modulating the expression of a serotonin receptor by ingesting a nutritional composition comprising specific amounts of (a) a lipid consisting of milk fat globule, (b) a lactoferrin, (c) prebiotics consisting of polydextrose and galactooligosaccharide, and it investigates anxiety-related behaviors after administrating it to rats after weaning.

Patent Documents 1 to 4, however, does not suggest it is capable of preventing the anxiety-related behavior in the growing stage in future which is attributable to the stress in infancy by ingesting the lactoferrin and/or the lactoferrin degradation product in infancy

For example, in the experimental model described in Patent Document 3, effectiveness of the lactoferrin in NEC model was investigated using premature animals obtained by cesarean section before scheduled delivery.

The treatment subject in the present technology is, however, the normal infant at birth, and thus the treatment subject is different from the experimental model in Patent Document 3 as compared. The experimental model in Patent Document 3 only showed a reduction in the incidence of NEC, necrotizing enterocolitis, during the treatment period, without any observation over time once the treatment was completed.

For example in experimental model described in Patent Document 4, rats during a growing stage are treated with a mixture of the lactoferrin, polydextrose and galactooligosaccharide and the reduction in an anxiety-related behavior induced by tail shocks is investigated.

In the present technology, however, infancy is employed as a treatment period, and thus the treatment period is different from the experimental model in Patent Document 4 as compared.

It is generally considered that a food material is low side effect and low efficacy if it is safe to eat it every day. Also being supported by the results of the experiment in Non-Patent Document 3, Liu Y.W. et al., it is common to think that the food material can not prevent the anxiety-related behavior which may occur in future during the growing stage and which is attributable to the stress in infancy.

Thus, (1) the present technology can provide an agent for preventing an anxiety-related behavior in a growing stage attributable to a stress in infancy comprising a lactoferrin and/or a lactoferrin degradation product as active ingredient characterized in that the lactoferrin and/or the lactoferrin degradation product is used via ingestion during infancy for a normal infant at birth and receives the stress in infancy.

Also, (2) the present technology can provide a food composition comprising a lactoferrin and/or a lactoferrin degradation product as active ingredient for preventing an anxiety-related behavior in a growing stage attributable to a stress in infancy characterized in that the lactoferrin and/or the lactoferrin degradation product is used via ingestion during infancy for a normal infant at birth and receives the stress in infancy.

Furthermore, (3) the present technology can provide a method for preventing an anxiety-related behavior in a growing stage attributable to a stress in infancy by administration of a lactoferrin and/or a lactoferrin degradation product as active ingredient characterized in that the lactoferrin and/or the lactoferrin degradation product is allowed to be ingested during infancy for a normal infant at birth and receives the stress in infancy.

Furthermore, (4) the present technology can provide a lactoferrin and/or a lactoferrin degradation product for preventing an anxiety-related behavior in a growing stage attributable to a stress in infancy or use thereof; characterized in that the aforementioned lactoferrin and/or a lactoferrin degradation product is used via ingestion during infancy for a normal infant at birth and receives the stress in infancy.

Furthermore, (5) the present technology can provide use of a lactoferrin and/or a lactoferrin degradation product into the aforementioned agent for preventing an anxiety-related behavior in a growing stage attributable to a stress in infancy or the aforementioned food composition for preventing an anxiety-related behavior in a growing stage attributable to a stress in infancy.

Furthermore, (6) the present technology can provide use of a lactoferrin and/or a lactoferrin degradation product for producing the aforementioned agent for preventing an anxiety-related behavior in a growing stage attributable to a stress in infancy or the aforementioned food composition for preventing an anxiety-related behavior in a growing stage attributable to a stress in infancy.

Furthermore, (7) the present technology can provide a method for producing a food composition for preventing an anxiety-related behavior in a growing stage attributable to a stress in infancy, the food composition comprising a lactoferrin and/or a lactoferrin degradation product as active ingredient, the method for producing the food composition being characterized in that the lactoferrin and/or the lactoferrin degradation product is used via ingestion during infancy for a normal infant at birth and receives the stress in infancy.

In the present technology, when the stress is a breakup of mother-child and father-child relationships, the anxiety-related behavior in the growing stage attributable to the stress in infancy can be prevented.

In the present technology, when the breakup of mother-child and father-child relationships is maternal separation, the anxiety-related behavior in the growing stage attributable to a stress in infancy can be prevented.

In the present technology, when the normal infant at birth is normal born after a gestational age of 37 weeks or more and less than 42 weeks, the anxiety-related behavior in the growing stage attributable to the stress in infancy can be prevented.

Advantageous Effects of Invention

According to the present technology, the agent for preventing the anxiety-related behavior in the growing stage attributable to the stress in infancy via ingestion of the lactoferrin and/or the lactoferrin degradation product for the normal infant at birth and receives the stress in infancy can be provided. In addition, the lactoferrin and/or the lactoferrin degradation product is highly safe to the infant because it is utilized as a food material.

The effects described here are not limitative and any effect described in the present technology may be contemplated.

Description of Embodiments

The followings describe the preferred embodiments for implementing the present technology. The embodiments described below are only examples of the representative embodiments of the present technology, and do not intend to allow the scope of the present technology to be interpreted narrowly.

The present technology comprises (a) and (b) shown below:

(a) capability of preventing possibility of the anxiety-related behavior in the growing stage attributable to the stress in infancy by using the lactoferrin and/or the lactoferrin degradation product via ingestion during infancy for the normal infant at birth and receives the stress in infancy; and,
(b) the agent for preventing the anxiety-related behavior in the growing stage attributable to the stress in infancy comprising the lactoferrin and/or the lactoferrin degradation product as active ingredient.

As used in the present technology, a "normal infant at birth" refers to a "neonate regarded generally as being healthy at birth".

More specifically, it refers to a "term-delivered infant who shows no abnormality during the gestation period and at the time of delivery".

Further specifically, it refers to a "neonate who exhibits no intrauterine fetal growth retardation during the gestation period and no abnormality, for example hypoxic ischemic encephalopathy, at the time of delivery".

Since the normal infant at birth and a neonate exhibiting intrauterine fetal growth retardation are different from each other in the central nervous system development process, and also since the present technology did not verify the preventive effects on the neonate exhibiting the intrauterine fetal growth retardation and assumption is also difficult due to the nature of the present technology, a "neonate exhibiting intrauterine fetal growth retardation during the gestation period " is excluded from the prevention subject of the present technology.

In the nervous system, morphologies and functions of neurogenesis, gliogenesis, synaptogenesis, myelinogenesis, synaptic pruning and the like exhibit dynamic changes over a prolonged period from an early stage of the fetal period through the time after birth in Non-Patent Document 4, Semple B D et al. Depending on the time of onset, the site and the degree of a disorder in the central nerve, the subsequent effects on the brain will vary.

It was reported that the intrauterine fetal growth retardation is at risk for onset of disorders such as learning disability and memory disorder and language disorder in Non-Patent Document 5, Geva R et al., and Non-Patent Document 6, Cosmi E et al. These disorders attributable to retarded development are classified as neurodevelopmental disorders, DSM-5, and diagnosed as autism spectrum disorder, attention-deficit / hyperactivity disorder, intellectual disabilities and the like.

In the present technology, the "normal infant at birth" is preferably a "term-delivered infant". As used herein, the "term delivery" of the "term-delivered infant" refers to a "delivery during 5 weeks from Day 0 of 37th week to Day 6 of 41st week of the gestation", and a "neonate born during this period " is referred to as a "term-delivered infant".

In the present technology, the "normal infant at birth" is preferably "normal born after a gestational age of 37 weeks or more and less than 42 weeks".

In the present technology, the "normal infant at birth" is more preferably a "term-delivered infant delivered within the range of a normal birth weight". A "body weight" which is "within the range of a normal delivery weight" refers to "2500 g or more and less than 4000 g in general".

In the present technology, the "stress in infancy" refers to a condition of exposure to an environmental factor by which the development of an infant is affected adversely, preferably refers to a "stress causing the lack of a sense, basic trust, of being loved and protected by surrounding humans, for example guardians and childcare givers".

As used herein, the "infantile stage" refers to "0, time of birth, to 3 years old".

As used herein, the "stress in infancy" includes the breakup of mother-child and father-child relationships.

The "mother-child and father-child" of the aforementioned "breakup of mother-child and father-child relationships" means a "guardian" or a "childcare giver", who may not only be father and mother but also be grandfather, grandmother and a third party as long as they are guardians or childcare givers.

The "guardian of the infant" in the present technology refers to a "person who supports the infant economically and raises the infant". The "childcare giver of the infant" in the present technology refers to a "person who cares the infant" including a" professional human such as a nurse and a babysitter other than parents, grandfathers and grandmothers".

In the "breakup of mother-child and father-child relationships", a cause of the breakup includes discord or defect of mother-child and father-child, mainly mother-child, relationship in the infant and discord or defect of familial relationship.

The "discord or defect of mother-child and father-child, mainly mother-child, relationship in the infant" includes maternal separation, neglect of the infant, abandonment of a child, child neglect or abandonment of custody of the infant, physical abuse of the infant, and psychological abuse of the infant. In the present technology, maternal separation is subjected preferably.

The "maternal separation" refers to a condition of "mother-child and father-child" separation and may include the "neglect of the infant". One which is subjected more preferably is a "condition in which neglect is not applicable but a good contact is poorly experienced because of "mother-child and father-child" separation" as a narrowly interpreted "maternal separation".

The "discord or defect of familial relationship" includes intra-familial divorce, intra-familial violence, intra-familial disease, postpartum depression, those resulting from economic distress, parent divorce, domestic violence and prolonged absence of parents.

In a subject for ingestion in the present technology, an infant who receives the stress continuously or intermittently during infancy is preferable.

As a "period during which the stress is received in infancy" in the present technology, an infant who receives the stress for at least one hour per day is subjected preferably, and an infant who receives a stress for 2 to 8 hours per day being more preferred and an infant who receives a stress for 2 to 4 hours per day being especially preferred.

For "detection of the stress in infancy", it is possible to make a judgment by asking the guardian or a relating person whether the "breakup of mother-child and father-child relationships" is applicable or not.

Also for detection of the stress in infancy, an infant who showed an increased blood stress hormone, cortisol, when compared with the standard level in infants can be judged to be an infant receiving the stress. For example, when it increases about twice or more compared with the standard level, it may be judged as infant received the stress.

An "ingestion period" in the present technology is preferably a long period within infancy because "the lactoferrin and/or the lactoferrin degradation product" of the present technology is highly safety and can give an enhanced preventive effect.

The "ingestion period" in the present technology is preferably a period from 0, after birth, to 3 years old for the purpose of increasing the preventive effect. Also when taking the ingestion period of a general formulated milk powder for child care into consideration, the "ingestion period" in the present technology is preferably 0, after birth, to 2 years, more preferably 0, after birth, to 1 year.

The time when the ingestion of "the lactoferrin and/or the lactoferrin degradation product" of the present technology is started may be the time when it is judged that the stress is given during infancy based on the aforementioned judgment procedure.

An "ingestion amount" in the present technology can appropriately be selected based on sex, ingestion interval and the like.

The "ingestion amount" in the present technology is preferably 0.01 to 1000 mg/kg body weight/day, more preferably 30 to 1000 mg/kg body weight/day, further preferably 50 to 1000 mg/kg body weight/day.

The "ingestion interval" of the present technology is not limited particularly, and the ingestion may be conducted several times a day using divided doses. For example, the ingestion may be conducted at the time of milk feeding or at a certain time interval, every several hours.

Also when the infant ingests a product comprising "the lactoferrin and/or the lactoferrin degradation product", ingesting approximately 20 mg/kg body weight/day, it is desirable to ingest further 10 to 30 mg/kg body weight/day more than this.

A method for ingestion in the present technology may be any of oral administration and parenteral administration, and oral ingestion is preferred in view of infancy.

The "state of an agent of prevention" in the present technology may be any of liquid, fluid, semi-solid, or solid. Liquid or fluid is preferred, because oral ingestion is preferable for infants.

The "growing stage" in the "anxiety-related behavior in the growing stage attributable to the stress in infancy" in the present technology refers to "6 to 19 years old". In the present technology, preferably, it is considered to having the prevent effect to "the anxiety-related behavior of pubescent, 12 to 18 years old". The "anxiety" of the "anxiety-related behavior in the growing stage attributable to the stress in infancy" in the present technology refers generally to "one of emotions emerging in a person when the person cannot counteract to a fear which is not clearly objective".

The "anxiety-related behavior" in the present technology is a behavior for escaping from a stimulation or situation which triggers an anxiety via a psychological symptom such as a potent anxiety, irritation, fear, and strain; a physical symptoms such as panic, palpitation, short breath, fatigue, nausea, headache, tension headache, tachycardia, chest pain, stomach ache, achromasia, perspiration, trembling, insomnia and mydriasis. The escaping behavior includes a behavior such as an emotional instability, a fear of contacting humans and an isolation oneself from society, being a shut-in, and it means that social life is impaired.

The "anxiety-related behavior in the growing stage attributable to the stress in infancy" which is a "subject for prevention" in the present technology is not limited particularly as long as it is an "anxiety-related behavior which is reported to be an anxiety-related behavior appearing during the growing stage due to the stress in infancy".

The "anxiety-related behavior" which is the aforementioned "subject for prevention" includes a behavior resulting from the mental diseases (1) to (4) shown below in Non-Patent Document 7, Higgins DJ et al., Non-Patent Document 8, Heim C et al. and Non-Patent Document 9, Green JG et al..

The mental disease includes (1) Anxiety Disorders whose major symptom is anxiety, (2) anxiety-associated Bipolar and Related Disorders, (3) Depressive Disorders and (4) Trauma- and Stressor- Related Disorders.

In the present technology, these anxiety-related behaviors, for example onsets of the symptoms, can be prevented.

A substance employed as active ingredient in the "agent for preventing the anxiety-related behavior in the growing stage attributable to the stress in infancy" in the present technology includes the lactoferrin and/or the lactoferrin degradation product. Amongst these, the lactoferrin is preferable because of its excellent efficacy.

The "lactoferrin" in the present technology is an iron-binding glycoprotein which is present in milk, tear, saliva, blood and the like. The lactoferrin is contained in milks such as colostrum, transitional milk, normal milk and late lactation milk especially of mammals such as cattle, humans, sheep, goats, pigs, mice and horses. Among these, milks of cattle and human are preferable in view of contents, availability and safety.

The molecular weight of bovine lactoferrin is 86,000 daltons and that of human lactoferrin is 88,000 daltons, hereinafter indicated as "Da".

Those exemplified in the present technology include a lactoferrin separated from the milk and a processed material therefrom such as defatted milk, whey and the like by ordinary methods such as ion exchange chromatography, hereinafter referred to also as "separated lactoferrin"; apolactoferrin obtained by removing iron from the separated lactoferrin using an acid such as hydrochloric acid and citric acid; a metal-saturated lactoferrin obtained by chelating a separated lactoferrin with a metal such as iron, copper, zinc and manganese; and a lactoferrin saturated with a metal at various saturation degrees.

In the present technology, the lactoferrins are not limited to those derived from mammals, and include recombinant lactoferrins produced by gene-engineered microorganisms or animals; and chemically synthesized lactoferrins.

The lactoferrins may be non-glycosylated or glycosylated ones.

Among these, any lactoferrin may be used independently or in a mixture with two or more.

The lactoferrin may be one which is commercially available or may be prepared from a raw material such as milk. A commercially available lactoferrin, for example Morinaga Milk Industry Co., Ltd., may be exemplified because it is obtained easily. A method for preparing the separated lactoferrin is described in Patent Document 1.

The "lactoferrin degradation product" in the present technology is preferably a hydrolysate starting from the aforementioned "lactoferrin" in the present technology as the raw material. The raw material is preferably the lactoferrin derived from milks of cattle, human and the like in view of contents, availability, safety, and easiness in production. It is also possible to use a commercially available lactoferrin degradation product.

The average molecular weight of the lactoferrin degradation product is preferably 5,000 Da or less, more preferably 2,000 Da or less. The rate of degradation of the lactoferrin degradation product is preferably 20% or less, more preferably 15% or less.

<Method for calculating average molecular weights>

The average molecular weight of a lactoferrin hydrolysate in the present technology is calculated based on the concept of a number average molecular weight described below.

The number average molecular weight indicates the average value of a high molecular weight compound based on the following different index, as described in a Document, Ed. by The Society of Polymer Science, Japan, "Fundamentals of polymer science", pages 116 to 119, Tokyokagaku dojin KK, 1978.,.

Because the high molecular weight compound such as a protein hydrolysate is a heterogeneous substance and has molecular weight is distributed, the molecular weight of the protein hydrolysate should be indicated as an average molecular weight for the purpose of physicochemical handling. The number average molecular weight, hereinafter abbreviated sometimes as Mn, is the average of the number of molecules, which is defined by the equation shown below when the molecular weight of peptide chain i is Mi and the number of the molecules is Ni:

[Equation 1]

$$Mn = \sum_{i=1}^{\infty} Mi\, Ni \Big/ \sum_{i=1}^{\infty} Ni$$

A method for producing the "lactoferrin degradation product" in the present technology may for example be, but is not limited to, the method described below (see Patent Document 2).

To a water dispersion comprising a lactoferrin as a starting material, a protein degradation enzyme is added to degrade the lactoferrin, and then after quenching an enzymatic reaction separation and purification for the resultant solution are conducted appropriately to obtain the lactoferrin degradation product.

It is usually preferable to adjust the water dispersion comprising the lactoferrin a lactoferrin concentration of about 3 to 15% by mass as protein conversion.

It is preferable to adjust the pH of the water dispersion comprising the lactoferrin to the optimum pH of the protein degradation enzyme employed, for example 2 to 4.

The protein degradation enzyme is preferably, but is not limited to, an enzyme having a lactoferrin degrading activity.

The protein degradation enzyme includes protease, tripepsin, chymotrypsin, plasmin, pepsin, papain, peptidase and aminopeptidase.

They may be either those derived from bacteria or those derived from animals or plants.

Those derived from bacteria include genera of *Bacillus, Bifidobacterium, Enterococcus, Lactobacillus, Lactococcus, Leuconostoc, Pediococcus, Propionbacter, Pseudomonas, Streptococcus,* or mixtures thereof.

Those derived from animals include gastric juice-derived pepsins such as sheep, goats, pigs, mice, water buffalos, camels, yaks, horses, donkeys, llamas, cattle and humans.

Any of these protein degradation enzymes may be used independently or in a mixture with two or more.

The used amount of the protein degradation enzyme may vary depending on the concentration of the starting materials, the potency of the enzyme, the reaction temperature, the reaction time, and the like. In general, 1000 to 20000 units, unit of activity, per gram of the lactoferrin as protein is desirable.

During the enzymatic reaction, the temperature of the reaction system is selected, not limited particularly, from a practical range including an optimal temperature range at which the action of an enzyme is expressed, and usually selected preferably within the range of 30 to 60°C. The reaction time is preferably 2 to 10 hours.

It is difficult to completely specify the duration of the reaction because a progressing state of the reaction may vary depending on the reaction condition such as reaction temperature and initial pH, and for example there may be a possible problem that the resultant degradation products having physicochemical properties which vary with production batches is generated if the duration of the reaction of the enzymatic reaction is set constant then. Accordingly, it is desirable to determine the duration of the reaction to achieve the desired value by monitoring the enzymatic reaction.

The method for monitoring the enzymatic reaction includes a method sampling a part of the reaction solution and measuring the rate of protein degradation and the average molecular weight of the proteins.

<Method for calculating rate of protein degradation>

In a method for calculating the rate of protein degradation, the total amount of nitrogen of a sample is measured by Kjeldahl method, Ed. by Japan Society for Food Engineering, "Food analysis method ", page 102, Korin KK, 1984, and the amount of formol nitrogen of the sample is measured by a formol titration method, Ed. by Mitsuda et al, "Experiments in food science and technology", 1st vol., page 547, Yokendo Co.,Ltd, 1970,, and then the resultant measured values are used to calculate the rate of degradation based on the equation shown below.

$$\text{Rate of Degradation } (\%) = (\text{the amount of formol nitrogen/the total amount of nitrogen}) \times 100$$

A quenching the enzymatic reaction is conducted by an ordinary method such as heat inactivation.

It is preferred, after inactivation, to conduct ordinary separation and purification. The method for separation and purification includes filtration, precision filtration, membrane separation and resin adsorption. Any of these may be conducted independently or in combination with two or more. During the separation and purification, the enzyme used can be removed.

When utilizing "the lactoferrin and/or the lactoferrin degradation product" in a pharmaceutical product, the pharmaceutical product may be for oral administration or parenteral administration and oral administration is preferred. The parenteral administration includes injection into blood, skin, muscle or the like, rectal administration and inhalation. A dosage form for the oral administration includes tablets, capsules, troches, syrups, granule formulations, powder formulations and ointments.

When formulating, a component such as excipients, pH modifiers, colorants, and taste masking agents which is employed usually for formulations may be employed in addition to "the lactoferrin and/or the lactoferrin degradation product". A component having efficacy for preventing or treating any diseases which are known or will be found in future may be used concomitantly depending on a purpose.

It is capable of formulating into desired dosage forms appropriately depending on the administration mode. For example, in the case of oral administration, it is capable of formulating solid formulations such as powder formulations, granule formulations, tablets and capsules; liquid formulations such as solution formulations, syrups, suspensions and emulsions. In the case of parenteral administration, it is capable of formulating formulation suppositories, spray formulations, ointments, patches and injection formulations.

The formulating may also be implemented by any appropriate known method depending on the dosage form. When formulating, only the casein-treated material or only the separated and purified fraction may be formulated, or appropriately it may be formulated by incorporating a formulation carrier.

The formulation carrier may be any of various organic or inorganic carriers depending on dosage forms. The carrier of a solid formulation includes excipient, binder, disintegrant, lubricant, stabilizers and flavoring agent.

Examples of the excipients include saccharide derivatives such as lactose, sucrose, glucose, mannitol and sorbitol; starch derivatives such as corn starch, potato starch, α-starch, dextrin and carboxymethyl starch; cellulose derivatives such as crystalline cellulose, hydroxypropyl cellulose, hydroxypropyl methyl cellulose, carboxymethyl cellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; and sulfate derivatives such as calcium sulfate.

Examples of the binders include gelatin; polyvinyl pyrrolidone; and Macrogol, in addition to any of the excipients described above.

Examples of the disintegrants include chemically modified starches or cellulose derivatives such as croscarmellose sodium, carboxymethyl starch sodium, and crosslinked polyvinyl pyrrolidone in addition to any of the excipients described above.

Examples of the lubricants include talc; stearic acid; metal stearates such as calcium stearate and magnesium stearate; colloidal silica; waxes such as veegum and spermaceti; boric acid; glycol; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acids such as silicic anhydride and silicic hydrate; and starch derivatives.

Examples of the stabilizers include p-hydroxybenzoates such as methyl paraben and propyl paraben; alcohols such as chlorobutanol, benzyl alcohol, and phenylethyl alcohol; benzalkonium chloride; acetic anhydride; and sorbic acid.

Examples of the flavoring agents include sweeteners, acidifiers and flavors.

Examples of the carriers employed in a liquid formulation for oral administration include solvent such as water and flavoring agents.

When "the lactoferrin and/or the lactoferrin degradation product" is utilized in foods and beverages for human or animals, it is capable of preparing them by adding it to known foods and beverages, or it is capable of producing new foods and beverages by mixing it with the raw materials for food and beverage.

The food and beverage includes milk and a dairy product regardless of the forms such as liquids, pastes, solids and powders.

The foods and beverages defined in the present technology may also be provided or sold as foods and beverages claimed health use.

The action of "claim" includes every action for making a consumer to be informed of the use. If it is an expression which can make the consumer recall or guess the use, all action of "claim" correspond to the action of "claim" of the present technology regardless of the purpose of the claiming, the contents of the claiming or the claimed subjects or media.

The "claim" is preferably implemented via an expression by which the consumer can recognize the use directly. It includes an activity to assign, deliver, display for the purpose of assignment or delivery and import a good or good a package relating to foods and beverages product which the use is described on; an activity to display or distribute advertisement materials, price lists or transaction documents relating to goods which the use is described on; or to provide such a detailed information which the use described on via an electromagnetic method such as internet.

Meanwhile, a content of claim is preferably a claim authorized by the relevant government, for example the claim authorized under various regulations prescribed by the government and implemented in a manner based on such an authorization. The content of claim is preferably attached to the advertising materials at the sites of selling such as packages, containers, catalogs, pamphlets and POPs and other documents.

The "claim" also includes a claim such as health foods, functional foods, enteral nutrition foods, foods for special use, foods with health claims, foods for specified health uses, nutritional functional foods and quasi-drugs. Among these, one which may especially be exemplified is a claim authorized by Consumer Affairs Agency, for example, a claim authorized under the regulations relating to food for specified health uses or analogous regulations. Examples of the latter include a claim as a food for specified health uses, a claim as a conditional food for specified health uses, a claim indicating an effect on body structure or function, and a disease risk reduction claim. More specifically, a claim as a food for specified health uses, especially health use claim, prescribed under the Ordinance for Enforcement of Health Promotion Act, Ordinance of the Ministry of Health, Labour and Welfare No.86 dated April 30, 2003, and analogous claims are exemplified typically.

When utilizing "the lactoferrin and/or the lactoferrin degradation product" in feeds, it is capable of preparing them by adding it to any of known feeds such as milks and dairy products or it is capable of producing new feeds by mixing it with the raw materials for the feed.

Accordingly, "the lactoferrin and/or the lactoferrin degradation product" of the present technology may be used as the food composition for preventing the anxiety-related behavior in the growing stage attributable to the stress in infancy and the like, or can be used as being contained in such a food composition.

Also, "the lactoferrin and/or the lactoferrin degradation product" of the present technology can be used for a method for preventing, ameliorating or treating various diseases, including underlying diseases, relating to or presumably relating to the anxiety-related behavior in the growing stage attributable to the stress in infancy, can be used as a component for preventing these various diseases, can be used as a component contained in a drug or a food for preventing these various diseases or can be used for producing such a drug or a food for preventing these various diseases.

As discussed above, the present technology can be utilized in the wide fields such as pharmaceuticals, foods, compositions for foods, dermal formulations and functional foods.

The present technology can also employ the following constitutions.

[1] An agent or a method for preventing an anxiety-related behavior in a growing stage attributable to a stress in infancy comprising a lactoferrin and/or a lactoferrin degradation product as active ingredient characterized in that the lactoferrin and/or the lactoferrin degradation product is used via ingestion during infancy for a normal infant at birth and receives the stress in infancy.

[2] The agent or the method for preventing the anxiety-related behavior in the growing stage attributable to the stress in infancy according to [1] wherein the stress is a breakup of mother-child and father-child relationships.

[3] The agent or the method according to [2] wherein the breakup of mother-child and father-child relationships is maternal separation.

[4] The agent or the method according to any one of [1] to [3] wherein a normal infant at birth is normal born after a gestational age of 37 weeks or more and less than 42 weeks.

[5] A lactoferrin and/or a lactoferrin degradation product for preventing the anxiety-related behavior in the growing stage attributable to the stress in infancy according to any one of [1] to [4].

[6] Use of a lactoferrin and/or a lactoferrin degradation product into an agent for preventing the anxiety-related behavior in the growing stage attributable to the stress in infancy according to any one of [1] to [4].

[7] Use of a lactoferrin and/or a lactoferrin degradation product for producing an agent for preventing the anxiety-related behavior in the growing stage attributable to the stress in infancy according to any one of [1] to [4].

[8] A food composition comprising a lactoferrin and/or a lactoferrin degradation product as active ingredient for preventing an anxiety-related behavior in a growing stage attributable to a stress in infancy and a method of producing it characterized in that the lactoferrin and/or the lactoferrin degradation product is used via ingestion during infancy for a normal infant at birth and receives stress in infancy.

Examples

In the following Examples, the present technology is further detailed. The following Examples are representatives of the present technology, by which the scope of the present technology is not interpreted narrowly.

<Method for producing lactoferrin and lactoferrin degradation product >

(1) As a lactoferrin, a commercial product extracted from bovine milk manufactured by Morinaga Milk Industry Co., Ltd. was employed.

(2) Production Example 1

A bovine lactoferrin manufactured by Morinaga Milk Industry Co., Ltd. was dissolved in a purified water at 5% by mass, and a resultant solution was adjusted at pH3 using a hydrochloric acid solution. The lactoferrin preparation obtained was warmed at 45°C and then a pepsin derived from a gastric juice at 3% based on the mass of the lactoferrin was added therein, and reacted for 6 hours with stirring thereby hydrolyzing.

After completion of the hydrolysis reaction, a resultant reaction solution was heated to 80°C at which it was kept for 10 minutes thereby inactivating the enzyme. After cooling a resultant solution, a sodium hydroxide solution was added to adjust the reaction fluid at pH 6, and thereafter a resultant reaction fluid was lyophilized to obtain a lactoferrin hydrolysate. An average molecular weight of the lactoferrin hydrolysate obtained is 1,600Da measured by the aforementioned <Method for calculating average molecular weights>. The degradation rate of the lactoferrin hydrolysate was 10% measured by <Method for calculating protein degradation rate>.

<Maternal separation model experiment>

Using a maternal separation model, it was investigated whether ingestion of each of the lactoferrin and the lactoferrin degradation product during infancy inhibits the anxiety-related behavior induced by a maternal separation.

The schedule of the experimental design of the maternal separation model experiment is shown in Table 1.

[Table 1]

Table 1

| Experiment design | | |
|---|---|---|
| Birth | | Weaning | |
| 5-Day old | | 21-Day old (3-Week old) | 38,39-Day old (5-Week old) |

(continued)

| Experiment design | | | | |
|---|---|---|---|---|
| Birth | | | Weaning | |
| | Maternal separation (MS) [3h/day, 5- to 21-day old] 5- to 15-day old: 30°C, 16- to 21-day old: 28°C | | Normal rearing | Elevated plus maze test |
| | Oral administration [5- to 21-day old], Test substance 300 mg/kgBW/day | | | |
| Group design | | | | |
| ♦ Non-treatment group: | No maternal separation, | | No treatment | |
| ♦ Treatment with physiological saline group: | Maternal separation, | | Treatment with physiological saline | |
| ♦ Lactoferrin treatment group: | Maternal separation, | | Treatment with lactoferrin | |
| ♦ Lactoferrin degradation product treatment group: | Maternal separation, | | Treatment with lactoferrin degradation product | |

Sprague Dawley, SD,-strain pregnant rats were purchased. A neonate which gave birth naturally was adjusted to 10 neonates, 5 males and 5 females, per a litter on the day next to the birth.

Over 3 hours a day during a period from the 5th to 21st day after birth, each of the newborn rats, males, was isolated from a maternal animal, the newborn rats of the same litter and placed an individual cylindrical container from a rearing cage. In order to prevent the lowering of the body temperature, the temperature of the floor was kept at 30°C during a period of 5- to 15-day old age and at 28°C during a period of 16- to 21-day old age. The newborn rats, females, of the same litter were not separated from the maternal animal and it was careful of not giving a stress to the maternal animal which was separated the newborn rats.

4 groups were provided including non-treatment group, maternal separation + physiological saline group, control group, maternal separation + lactoferrin treatment group, lactoferrin group, maternal separation + lactoferrin degradation product treatment group, lactoferrin degradation product group.

In non-treatment group, newborn rats which were not subjected to the maternal separation and the treatment and were raised normally were employed. In maternal separation group, 3 maternal separation groups were pooled and assigned to 5 males per litter in order to avoid any effects of difference between individual maternal animals.

In non-treatment group, the newborn rats were not subjected to the maternal separation and were not treated with any of the lactoferrin or the lactoferrin degradation product.

In lactoferrin group, the newborn rats were treated via a sonde with the lactoferrin (300 mg/kg/day) via a sonde at the same time as the maternal separation.

In lactoferrin degradation product group, the lactoferrin degradation product (300 mg/kg/day) was treated at the same time as the maternal separation.

In control group, the newborn rats were treated with a physiological saline instead of the lactoferrin and the lactoferrin degradation product at the same time as the maternal separation.

After weaning at 21-day old age in all 4 groups, each 2 male rats were housed in one cage and were reared under an ordinary feed and drinking water.

At 38- and 39-day old age, the anxiety-related behavior in the rats was monitored using an elevated plus maze. The elevated plus maze test measures the anxiety-related behavior in the rats and are utilized frequently in pharmacological tests and the like. A plus-shaped platform located at about 1 meter above the floor consists of two arms facing each other which have walls, closed arms, and two arms which have no walls, open arms. A rat placed on the center tends to avoid entering into the open arms and to prefer to enter into the closed arms if it has an enhanced fear of a high place.

In this test, travel distances and the number of arm entries of the rats were recorded during a test period of 5 minutes, and the rate of entry into each arm, the number of entries into each arm/total number of entries × 100, and the travel distance were used as indices of the anxiety.

In the model of the maternal separation during a lactating stage, a blood level of a stress hormone, corticosterone, at 10-day old age were 11.66 ng/mL in "non-treatment group" and 20.11 ng/mL in "maternal separation + physiological saline group", observing a difference by about 2 times in "maternal separation + physiological saline group" compared with "non-treatment group".

The corticosterone is a stress hormone of a rodent, which corresponds to cortisol in humans.

Table 2 shows that the results of the maternal separation model experiment.

When the control group was compared with non-treatment group, the maternal separation during the lactating stage caused a reduction in the rate of entry into the open arms and an increase in the rate of entry into the closed arms, which reflected the induction of the anxiety-related behavior (P<0.05, t-test).

The rat treated orally with at least one of the lactoferrin or the lactoferrin degradation product during the lactating stage exhibited no reduction in the rate of entry into the open arms and it was on the same level as non-treatment group.

A total travel distance significantly reduce due to the maternal separation caused a significant reduction, but the total travel distance improved due to the treatment with at least any of the lactoferrin or the lactoferrin degradation product caused amelioration.

Accordingly, it was shown that the treatment with at least any of the lactoferrin or the lactoferrin degradation product during the lactating stage was capable of inhibiting the growing stage anxiety-related behavior induced by the maternal separation.

[Table 2]

Table 2

| Group | % Entry into open arms | % Entry into closed arms | Total travel distance (cm) |
|---|---|---|---|
| Non-treatment | 11.44 ± 2.17 | 38.32 ± 2.16 | 3107 ± 120 |
| Maternal separation + physiological saline | 2.86 ± 2.86* | 47.14 ± 2.86* | 1989 ± 195* |
| Maternal separation + Lactoferrin | 11.02 ± 4.89 | 38.98 ± 4.89 | 2656 ± 308 |
| Maternal separation + Lactoferrin degradation product | 11.88 ± 10.97 | 38.12 ± 10.97 | 2827 ± 75 |
| *p<0.05 (vs non-treatment group, t-test) | | | |

A requirement for extrapolation of the maternal separation condition of the maternal separation model experiment to humans are shown in Table 3.

[Table 3]

Table 3

| Rats | Corresponding factor | Humans |
|---|---|---|
| 5-Day old | <Maternal separation (treatment) starting period> | At birth (term-delivered infant) |
| 21-Day old | <Maternal separation (treatment) completing period> | 2 to 3 years old |
| 300mg/kg/day | <Dosage> | 3mg/kg/day |
| 38,39-Day old (5-Week old) | <Time of anxiety-related behavior> | Growing stage (6 to 19 years old) |
| Reduction in % entry into open arms | <Type of behavior> | <Growing stage anxiety-related behavior> |

It is shown that the administration of the lactoferrin or the lactoferrin degradation product during the human infancy inhibits the maternal separation-induced anxiety-related behavior in growing stage based on the requirements for extrapolation to humans in Table 3.

Accordingly, it was shown that the anxiety-related behavior in the growing stage attributable to the stress in infancy can be prevented using the lactoferrin and/or the lactoferrin degradation product via ingestion during infancy for a normal infant at birth and receives a stress in infancy.

**Claims**

1. An agent for preventing an anxiety-related behavior in a growing stage attributable to a stress in infancy comprising a lactoferrin and/or a lactoferrin degradation product as active ingredient **characterized in that** the lactoferrin and/or the lactoferrin degradation product is used via ingestion during infancy for a normal infant at birth and receives the stress in infancy.

2. The agent for preventing the anxiety-related behavior in the growing stage attributable to the stress in infancy according to Claim 1 wherein the stress is a breakup of mother-child and father-child relationships.

3. The agent according to Claim 2 wherein the breakup of mother-child and father-child relationships is maternal separation.

4. The agent according to any one of Claims 1 to 3 wherein the normal infant at birth is normal born after a gestational age of 37 weeks or more and less than 42 weeks.

5. A food composition comprising a lactoferrin and/or a lactoferrin degradation product as active ingredient for preventing an anxiety-related behavior in a growing stage attributable to a stress in infancy **characterized in that** the lactoferrin and/or the lactoferrin degradation product is used via ingestion during infancy for a normal infant at birth and receives the stress in infancy.

6. A method for preventing an anxiety-related behavior in a growing stage attributable to a stress in infancy by administration of a lactoferrin and/or a lactoferrin degradation product as active ingredient **characterized in that** the lactoferrin and/or the lactoferrin degradation product is allowed to be ingested during infancy for a normal infant at birth and receives the stress in infancy.

7. The method for preventing the anxiety-related behavior in the growing stage attributable to the stress in infancy according to Claim 6 wherein the stress is a breakup of mother-child and father-child relationships.

8. The method for prevention according to Claim 7 wherein the breakup of mother-child and father-child relationships is maternal separation.

9. The method for prevention according to Claim 6 wherein the normal infant at birth is normal born after a gestational age of 37 weeks or more and less than 42 weeks.

10. The method for prevention according to Claim 6 wherein the anxiety-related behavior is selected from the group consisting of anxiety disorder, bipolar disorder, depressive disorder, trauma- and stressor-related disorder.

11. Use of a lactoferrin and/or a lactoferrin degradation product for preventing the anxiety-related behavior in the growing stage attributable to the stress in infancy according to any one of Claims 6 to 10.

12. Use of a lactoferrin and/or a lactoferrin degradation product into the agent or the food composition for preventing the anxiety-related behavior in the growing stage attributable to the stress in infancy according to any one of Claims 1 to 5.

13. Use of a lactoferrin and/or a lactoferrin degradation product for producing the agent or the food composition for preventing the anxiety-related behavior in the growing stage attributable to the stress in infancy according to any one of Claims 1 to 5.

14. A method for producing a food composition for preventing an anxiety-related behavior in a growing stage attributable to a stress in infancy,
the food composition comprising a lactoferrin and/or a lactoferrin degradation product as active ingredient,
the method for producing the food composition being **characterized in that** the lactoferrin and/or the lactoferrin degradation product is used via ingestion during infancy for a normal infant at birth and receives the stress in infancy.

## INTERNATIONAL SEARCH REPORT

| | International application No. |
|---|---|
| | PCT/JP2017/038597 |

**A. CLASSIFICATION OF SUBJECT MATTER**
Int.Cl. A61K38/14(2006.01)i, A61P25/22(2006.01)i

According to International Patent Classification (IPC) or to both national classification and IPC

**B. FIELDS SEARCHED**

Minimum documentation searched (classification system followed by classification symbols)
Int.Cl. A61K38/14, A61P25/22

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model applications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII), CAplus/MEDLINE/EMBASE/BIOSIS (STN), DWPI (Thomson Innovation)

**C. DOCUMENTS CONSIDERED TO BE RELEVANT**

| Category* | Citation of document, with indication, where appropriate, of the relevant passages | Relevant to claim No. |
|---|---|---|
| X | 竹内崇ほか, 新生子ラットの長期母子分離による発育期不安関連行動に対するラクトフェリンの効果, 日本獣医学会学術集会講演要旨集, 31 August 2005, vol. 140th, p. 193, ISSN 1347-8621, the whole document, (Japanese Society of Veterinary Science), non-official translation (TAKEUCHI, Takashi, et al., "Effect of lactoferrin on behaviors related to anxiety in development period due to long-term separation of newborn infant rat from parent rat") | 1-14 |
| X | 福万朋子ほか, 新生子期の母子分離ストレスによる発育期不安関連行動はラクトフェリンによって改善されるか, 日本獣医学会学術集会講演要旨集, 01 March 2005, vol. 139th, p. 181, ISSN 1347-8621, the whole document, (Japanese Society of Veterinary Science), non-official translation (FUKUMITSU, Tomoko, et al., "Anxiety in development period due to stress caused by parent-infant separation in neonatal period can be alleviated by lactoferrin?") | 1-14 |

☒ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 January 2018 (16.01.2018) | 30 January 2018 (30.01.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| | International application No. |
|---|---|
| | PCT/JP2017/038597 |

C (Continuation).   DOCUMENTS CONSIDERED TO BE RELEVANT

| Category* | Citation of document, with indication, where appropriate, of the relevant passages | Relevant to claim No. |
|---|---|---|
| A | 竹内崇ほか，ミルク由来ラクトフェリンは子ラットの分離不安を軽減する，日本獣医学会学術集会講演要旨集，  10 September 2003, vol. 136th, p. 228, ISSN 1347-8621, the whole document, (Japanese Society of Veterinary Science), non-official translation (TAKEUCHI, Takashi, et al., "Milk-derived lactoferrin alleviates separation anxiety of infant rat") | 1-14 |
| A | WO 2014/099134 A9 (MJN U.S. HOLDINGS LLC) 26 June 2014, claims & JP 2016-503025 A & US 2012/0171176 A1 & EP 2658388 A & CN 103327828 A | 1-14 |
| A | JP 2011-063552 A (HOKKAIDO UNIV.) 31 March 2011, paragraphs [0048]-[0053] (Family: none) | 1-14 |
| A | 和田剛ほか，ラットのストレス応答に対するウシミルク由来ラクトフェリンの効果，日本獣医学会学術集会講演要旨集，01 March 2005, vol. 139th, p. 182, ISSN 1347-8621, the whole document, (Japanese Society of Veterinary Science), non-official translation (WADA, Tsuyoshi, et al., "Effect of bovine milk-derived lactoferrin on stress response of rats") | 1-14 |
| A | 竹内崇ほか，ミルク由来ラクトフェリンによるストレス反応軽減効果，日本栄養·食糧学会総会講演要旨集，01 April 2003, vol. 57th, p. 279, ISSN 1347-9121, the whole document, , non-official translation (TAKEUCHI, Takashi, et al., "Effect of milk-derived lactoferrin on alleviation of stress response", Abstracts of the meeting, Japan Society of Nutrition and Food Science) | 1-14 |
| A | 亀森直ほか，ミルク由来ラクトフェリンによる成熟ラットのストレス反応軽減効果，日本獣医学会学術集会講演要旨集，10 September 2003, vol. 136th, p. 227, ISSN 1347-8621, the whole document, (Japanese Society of Veterinary Science), non-official translation (KAMEMORI, Nao, et al., "Effect of milk-derived lactoferrin on alleviation of stress response of adult rats") | 1-14 |
| A | WO 03/061688 A1 (NRL PHARMA, INC.) 31 July 2003, claims & US 2005/0119162 A1, claims & EP 1477182 A1 | 1-14 |
| A | JP 2007-106720 A (NAGASAKI UNIV.) 26 April 2007, claims (Family: none) | 1-14 |
| A | SACKER, A., et al., "Breast feeding and intergenerational social mobility: what are the mechanisms?", Arch. Dis. Child., 24 June 2013, vol. 98, no. 9, pp. 666-71, ISSN 1468-2044, p. 666 abstact | 1-14 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5427713 B
- JP 2016121109 A
- WO 2014099134 A
- US 20150119322 A

**Non-patent literature cited in the description**

- **HUOT R L et al.** *Psychopharmacology,* 2001, vol. 158, 366-373
- **TRUJILLO V et al.** *Stress,* 2016, vol. 19 (1), 91-103
- **LIU Y W et al.** *Brain Research,* 2016, vol. 1631, 1-12
- **SEMPLE B D et al.** *Prog Neurobiol,* 2013, vol. 106-107, 1-16
- **GEVA R et al.** *Pediatrics,* 2006, vol. 118 (1), 91-100
- **COSMI E et al.** *J Pregnancy,* 2011, 364381
- **HIGGINS D J et al.** *Aggress Violent Behav,* 2001, vol. 6 (6), 547-578
- **HEIM C et al.** *Biol Psychiatry,* 2001, vol. 49 (12), 1023-1039
- **GREEN J G et al.** *Arch Gen Psychiatry,* 2010, vol. 67 (2), 113-123
- Fundamentals of polymer science. Tokyokagaku dojin KK, 1978, 116-119
- Food analysis method. Korin KK, 1984, 102
- Experiments in food science and technology. Yoken-do Co.,Ltd, 1970, vol. 1, 547
- **LABOUR ; WELFARE.** *Ordinance for Enforcement of Health Promotion Act, Ordinance of the Ministry of Health,* 30 April 2003